# EUROPEAN PATENT SPECIFICATION

(11) **EP 0 868 427 B1**
(45) Date of publication and mention of the grant of the patent: **14.05.2003**
(21) Application number: 96944525.3
(22) Date of filing: 18.12.1996
(51) Int. Cl.: C07K 1/04, G01N 33/68, C12Q 1/68, C07K 14/00

(54) **METHODS FOR IDENTIFYING COMPOUNDS THAT BIND TO A TARGET**
VERFAHREN ZUM NACHWEIS VON VERBINDUNGEN DIE BESTIMMTE ZIELMOLEKÜLE BINDEN
PROCEDES D'IDENTIFICATION DE COMPOSES SE LIANT A UNE CIBLE

(30) Priority: 18.12.1995 US 573786
(43) Date of publication of application: 07.10.1998
(60) Divisional application: 02079205.7
(73) Proprietor: Praecis Pharmaceuticals Incorporated, Cambridge, MA 02139-1572 (US)
(72) Inventor: BENJAMIN, Howard, Lexington, MA 02173 (US); FINDEIS, Mark A., Cambridge, MA 02138 (US); GEFTER, Malcolm, L., Lincoln, MA 01773 (US); MUSSO, Gary, Hopkinton, MA 01748 (US); SIGNER, Ethan, R., Cambridge, MA 02140 (US)
(74) Representative: Price, Vincent Andrew
(86) International application number: US9620561
(87) International publication number: WO97022617

(56) References cited:
- EP-A- 0 639 584
- WO-A-90/02809
- WO-A-94/26775
- WO-A-95/16209
- WO-A-95/27072
- WO-A-96/41180
- PROCEEDINGS OF THE NATIONAL ACADEMY OF SCIENCES OF USA, vol. 87, 1990, WASHINGTON US, pages 6378-6382, XP000141872 S E CWIRLA ET AL.: "Peptides on phage: a vast library of peptides for identifying ligands" cited in the application
- CHEMICAL ABSTRACTS, vol. 120, no. , 28. February 1994, Columbus, Ohio, US, page , column , abstract no. 107692, V NIKOLAIEV ET AL.: "Peptide-encoding for structure determination of nonsequenceable polymers within libraires synthesized and tested on solid-phase supports"

## Description

### Background of the Invention

Recent advances in methods for producing large libraries of peptides have provided unprecedented numbers of peptides which can be screened for pharmaceutical activity. Both chemical and biological methods for synthesis of peptide libraries have been reported. For example, libraries of peptides (*e*.*g*., having 10⁶-10¹² member peptides) can be displayed on the surface of bacteriophage (known as "phage display" libraries). Such peptide libraries can comprise all possible peptides of a given length (*e.g*., every one of the twenty natural amino acid residues at each position of a hexamer), or a subset of all possible peptides. Methods for screening large libraries of peptides, to identify those peptides that bind to a target, have also been developed, such as biopanning. These screening techniques allow for the isolation from a library of one, or several, peptides that bind to a pre-selected target. By producing and screening large peptide libraries, it has become possible to rapidly search for peptides (*e.g*., ligands) that bind to a target (*e.g.,* a receptor). Moreover, the structure of selected peptides can be determined with relative ease by standard sequencing methodologies (*e.g*., sequencing of the peptides themselves or of a nucleic acid molecule encoding the peptide).

Despite the advantages of peptide libraries (*e.g*., immense diversity and simple "deconvolution" of the peptide structure by sequencing), the use of this approach to identify peptides that bind a target for pharmaceutical purposes has a number of drawbacks. For example, the affinity of a selected peptide(s) for the target often is relatively low (*e.g.*, high enough to detect binding of the peptide to the target but too low for pharmaceutical potency). Moreover, peptides are not always suitable for therapeutic administration due to such problems as difficulties in formulation (due to insolubility), unfavorable pharmacokinetics and/or pharmacodynamics, and rapid degradation *in vivo.*

Alternative to peptide libraries, libraries of non-peptide chemical compounds (*e.g*., peptidomimetics, peptide derivatives, peptide analogues, etc.) can be synthesized. Screening of a target with a non-peptide library may lead to the identification of a compound(s) with higher affinity for the target than that of a peptide selected by random peptide library screening and/or identification of a compound(s) with more desirable pharmacological properties than a peptide. However, the diversity of compounds that can be achieved by random chemical synthesis is considerably lower than that of random peptide library synthesis, thereby reducing the likelihood of identifying a high affinity target-binding compound from a randomly synthesized chemical library. An additional disadvantage of a chemical library approach to identifying molecules that bind a target is that determination of the structure of the compound(s) that binds the target (*i.e*., "deconvolution" of the compound structure) cannot be accomplished by a simple sequencing methodology but rather requires more complex chemical strategies, thereby limiting the number of identified compounds that can be efficiently analyzed.

Improved methods for identifying compounds that bind a target that retain the advantageous properties of both peptide library screening and chemical library screening while reducing or eliminating the disadvantageous properties of these techniques are needed.

EP-A-639584 discloses a method for preparing a polymer library, e.g., a peptide library, in which a highly diverse collection of peptides is synthesised on beads by solid phase peptide synthesis techniques. The library is then presented to potential targets and screened for the capacity to recognise and bind these targets. Selected library members are partially sequenced, and then rescreened in a reiterative manner until the binding polymers have been fully sequenced. The same type of library (e.g., a peptide library) is used in each iteration.

### Summary of the invention

The present invention features methods for identifying compounds that bind a target that combine the use of peptide-based libraries with the use of chemically-based libraries such that the advantages of each approach are maintained while many of the disadvantages of using either approach alone are overcome. For example, the methods of the invention provide the diversity and ease of deconvolution of traditional peptide library screening yet also provide for the identification of compounds with high affinity for the target and desirable pharmacological properties. To optimize the benefits of both peptide-based and chemically-based libraries, the methods of the invention involve utilizing information obtained from screening a target with a first library comprising a multiplicity of peptides in the design of a second library comprising a multiplicity of non-peptide compounds, as defined on page 2b. The target is then rescreened with this second library to identify compounds that bind to the target.

In accordance with one aspect of the invention, there is provided a method involving the following steps:
(a) forming a first library comprising a multiplicity of peptides, said first library being biologically generated;
(b) selecting from the first library at least one peptide that binds to the target;
(c) determining the sequence or sequences of the at least one peptide that binds to the target, thereby forming a peptide motif;
(d) forming a second library comprising a multiplicity of non-natural amino acid containing compounds designed based on the peptide motif;
(e) selecting from the second library at least one non-natural amino acid containing compound that binds to the target; and
(f) determining the structure or structures of the at least one non-natural amino acid containing compound that binds to the target;
thereby identifying a compound that binds to the target.

In another aspect of the invention, there is provided a method for identifying a compound that binds to a target, the method comprising:
(a) forming a first library comprising an anchor library of a multiplicity of peptides;
(b) selecting from the first library at least one peptide that binds to the target;
(c) determining the sequence or sequences of the at least one peptide that binds to the target, thereby generating a peptide motif;
(d) forming a second library comprising a multiplicity of non-natural amino acid containing compounds designed based on the peptide motif;
(e) selecting from the second library at least one non-natural amino acid containing compound that binds to the target; and
(f) determining the structure or structures of the at least one non-natural amino acid containing compound that binds to the target.

The term "non-natural amino acid containing compounds" as used herein is intended to include compounds comprising at least one molecule other than a natural amino acid residue wherein the structures of the compounds cannot be determined by standard sequencing methodologies bur rather must be determined by more complex strategies such as mass spectrometric methods.

The term "non-peptide compounds" as used hereinbelow is intended to have the same meaning as "non-natural amino acid containing compounds", and the two terms are used interchangeably in this application.

The first library is composed of peptides whose structures can be determined by standard sequencing methodologies (e.g. direct sequencing of the amino acids making up the peptides or sequencing of nucleic acid molecules encoding the peptide. Thus, the first library provides the extensive diversity of peptide libraries and the ease of deconvoluting the selected peptides. In contrast, the second, non-peptide library preferably comprises compounds that, while not peptides, are structurally related to peptides, such as peptide analogues, peptide derivatives and/or peptidomimetics. The structure of the non-peptide compounds preferably is determined by a mass spectrometric method, most preferably by tandem mass spectrometry. Since the second library is designed based on the peptide motif generated from screening the first library, many of the disadvantages of traditional chemical libraries (such as reduced diversity and more laborious deconvolution methods) are reduced or eliminated, since the second library is "biased" toward compounds that have affinity for the target. This bias in the second library for compounds having affinity for the target means that fewer compounds need to be screened as compared to a random chemically-synthesized library and, accordingly, fewer compounds need to be analyzed structurally (i.e. deconvoluted).

In a preferred embodiment, compounds identified by screening of the second library have at least 10-fold higher affinity for the target than the peptides identified by screening the first library. More preferably, compounds identified by screening of the second library have at least 100-fold higher affinity for the target than the peptides identified by screening the first library. Even more preferably, compounds identified by screening of the second library have at least 1000-fold higher affinity for the target than the peptides identified by screening the first library.

The methods of the invention can further involve additional library screening steps. For example, after compounds from the second library that bind the target have been identified, a third library can be formed that comprises a multiplicity of non-peptide compounds designed based on the structure or structures of the non-peptide compounds identified from the second library. The target can be rescreened with the third library to identify additional compounds that binds to the target.

Another aspect of the invention pertains to a library composed of a multiplicity of non-peptide compounds designed based on a peptide motif, wherein the peptide motif is determined by selecting from a peptide library at least one peptide that binds to a target, determining the sequence or sequences of the at least one peptide that binds to the target and determining a peptide motif.

### Brief Description of the Drawing

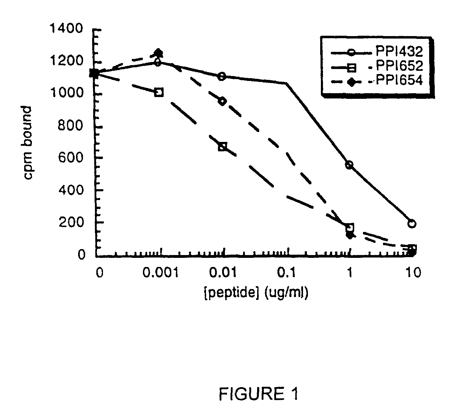

Figure 1 is a graph depicting the ability of compounds PPI-432. PPI-652 and PPI-654 to inhibit the binding of radiolabeled FGF to an anti-FGF antibody.

### Detailed Description of the Invention

The present invention pertains to methods for identifying a compound that binds to a target, as well compounds identified thereby, and libraries for use in the methods of the invention. The methods of the invention involve screening a target with at least two distinct libraries. The term "target", as used herein, is intended to include molecules or molecular complexes with which compounds (*e.g.*, peptides or non-peptide compounds) can bind or interact. Exemplary targets include ligands, receptors, hormones, cytokines, antibodies, antigens, enzymes, and the like. The target can be, for example, a purified compound or a partially purified compound or it can be associated with the surface of a cell that expresses the target.

In the methods of the invention, a target is initially screened with a peptide library to generate a peptide motif for peptides that can bind to the target. Accordingly, the methods of the invention first involve:
forming a first library comprising a multiplicity of peptides;
selecting from the first library at least one peptide that binds to the target; and
determining the sequence or sequences of the at least one peptide that binds to the target, thereby generating a peptide motif.

The term "peptides", as used herein with regard to libraries, is intended to include molecules comprised only of natural amino acid residues (*i.e*., alanine, arginine, aspartic acid, asparagine, cysteine, glutamic acid, glutamine, glycine, histidine, isoleucine, leucine, lysine. methionine, phenylalanine, proline, serine, threonine, tryptophan, tyrosine and valine) linked by peptide bonds, or other residues whose structures can be determined by standard sequencing methodologies (*e.g*., direct sequencing of the amino acids making up the peptides or sequencing of nucleic acid molecules encoding the peptide). The term "peptide" is not intended to include molecules structurally related to peptides. such as peptide derivatives, peptide analogues or peptidomimetics, whose structures cannot be determined by standard sequencing methodologies but rather must be determined by more complex chemical strategies, such as mass spectrometric methods.

The term "multiplicity", as used herein, refers to a plurality of different molecules (*e.g*., peptides or non-peptide compounds). Thus a "library comprising a multiplicity of peptides" refers to a library of peptides comprising at least two different peptide members. In preferred embodiments, libraries of peptides useful in the present invention include at least about 10³ different peptides, more preferably at least about 10⁶ different peptides and even more preferably at least about 10⁹ different peptides. Depending on the length of the peptide members and the efficiency of synthesis, library diversity as high as about 10¹² different peptides or even about 10¹⁵ different peptides may be achievable. A library comprising a multiplicity of peptides for use in the methods of the invention can comprise all possible peptides of a specified length (*i.e*., a "complete" random library wherein each position of the peptide can be any one of the twenty natural amino acid residues, *e.g*., all possible hexapeptides). Alternatively, a peptide library can include only a subset of all possible peptides of a specified length by having non-degenerate positions within the peptide library (*i.e*., one or more positions within the peptide which are occupied by only one, or a few, different amino acid residue(s) within each peptide member of the library). Moreover, as the peptide length increases, it may not be possible to achieve every possible peptide permutation within the library. Preferably, at least about 10⁵ to 10⁸ permutations of all possible permutations of a randomized peptide are present within the library. The length of the peptides used in the library can vary depending upon, for example, the degree of diversity desired and the particular target to be screened. For example, in different embodiments, the peptide library is made up of peptides not longer than about 30 amino acids long, not longer than about 20 amino acids long or not longer than about 12 amino acids long. Preferably, the peptide library is comprised of peptides at least 3 amino acids long. and more preferably at least 6 amino acids long.

A library comprising a multiplicity of peptides can be formed by any one of several methods known in the art. For example, in one embodiment, a multiplicity of nucleic acid molecules encoding a multiplicity of random peptides are synthesized and the nucleic acid molecules are introduced into a vector that allows for expression of the encoded peptide library. One examples of such a library is an "external" library in which the peptide library is expressed on a surface protein of a host, such as a "phage display" library (see, *e.g*., Smith, G.P. (1985) *Science* 228:1315-1317; Parmley, S.F. and Smith, G.P. (1988) *Gene* 73:305-318; and Cwirla, S. *et al*. (1990) *Proc. Natl. Acad. Sci. USA* 87:6378-6382). As used herein, a "phage display" library is intended to refer to a library in which a multiplicity of peptides is displayed on the surface of a bacteriophage, such as a filamentous phage, preferably by fusion to a coat protein of the phage (*e.g.,* the pIII protein or pVIII protein of filamentous phage). In phage-display methods, a multiplicity of nucleic acid molecules coding for peptides is synthesized and inserted into a phage vector to provide a recombinant vector. Suitable vectors for construction of phage display libraries include fUSE vectors, such as fUSE1, fUSE2, fUSE3 and fUSE5 (Smith and Scott (1993) *Methods Enzymol*. 217:228-257). Nucleic acid molecules can be synthesized according to methods known in the art (see, *e.g*., Cormack and Struhl, (1993) *Science* 262:244-248), including automated oligonucleotide synthesis . Following insertion of the nucleic acid molecules into the phage vector, the vector is introduced into a suitable host cell and the recombinant phage are expressed on the cell surface after a growth period. The recombinant phage can then be used in screening assays with a target (described further below).

Another example of a peptide library encoded by a multiplicity of nucleic acid molecules is an "internal" library, wherein the peptide members are expressed as fusions with an internal protein of a host (*i.e*., a non-surface protein) by inserting the nucleic acid molecules encoding the peptides into a gene encoding the internal protein. The internal protein may remain intracellular or may be secreted by, or recovered from. the host. Examples of internal proteins with which peptide library members can be fused include thioredoxin, staphnuclease, lac repressor (LacI), GAL4 and antibodies. An internal library vector is preferably a plasmid vector. In one example of an internal library. referred to as a two-hybrid system (see *e.g.,* U.S. Patent No. 5,283,173 by Field; Zervos *et al*. (1993) *Cell* 72:223-232; Madura *et al*. (1993) *J. Biol. Chem.* 268:12046-12054; Bartel *et al.* (1993) *Biotechniques* 14:920-924; and Iwabuchi *et al*. (1993) *Oncogene* 8:1693-1696), nucleic acid molecules encoding a multiplicity of peptides are inserted into a plasmid encoding the DNA binding domain of GAL4 (GAL4db) such that a library of GAL4db-peptide fusion proteins are encoded by the plasmid. Yeast cells (*e.g., Saccharomyces cerevisiae* YPB2 cells) are transformed simultaneously with the plasmid encoding the library of GAL4db-peptide fusion proteins and a second plasmid encoding a fusion protein composed of the target fused to the activation domain of GAL4 (GAL4ad). When the GAL4ad-target interacts with a GAL4db-peptide library member, the two domains of the GAL4 transcriptional activator protein are brought into sufficient proximity as to cause transcription of a reporter gene or a phenotypic marker gene whose expression is regulated by one or more GAL4 operators.

In another example of an internal library (see *e.g.,* U.S. Patents 5,270,181 and 5,292,646, both by McCoy), nucleic acid molecules encoding a multiplicity of peptides are inserted into a plasmid encoding thioredoxin such that a library of thioredoxin-peptide fusion proteins are encoded by the plasmid. The plasmid is introduced into a bacterial host cell where the thioredoxin-peptide fusion proteins are expressed cytoplasmically. The fusion proteins can be selectively released from the host cells (*e.g.*, by osmotic shock or freeze-thaw procedures) and recovered for use in screening assays with a target.

In yet another example of an internal library (described further in Cull, M.G. *et al.* (1992) *Proc. Natl. Acad. Sci. USA* 89:1865), nucleic acid molecules encoding a multiplicity of peptides are inserted into a gene encoding LacI to create a fusion gene encoding a fusion protein of LacI and the peptide library members. The plasmid encoding the fusion protein library members is designed such that the fusion proteins bind to the plasmid (*i.e.,* a plasmid encoding the LacI fusion proteins includes *lac* operator sequences to which LacI binds) such that the fusion proteins and the plasmids encoding them can be physically linked. Following expression of the fusion proteins in host cells, the cells are lysed to liberate the fusion protein and associated DNA, and the library is screened with an immobilized target. Fusion proteins that bind to the target are recovered and the associated DNA is reintroduced into cells for amplification and sequencing, thus allow for determination of the peptide sequence encoded by the DNA.

A particularly preferred peptide library for use in the methods of the invention is an anchor library as described in U.S. Patent Application Serial No. 08/479,660 (WO-A-9641180), entitled *Anchor Libraries and Identification of Peptide Binding Sequences*, and corresponding PCT Application No. PCT/US96/09383 (WO-A-9641180). As used herein, the term "anchor library" refers to a peptide library in which the peptides have non-continuous regions of random amino acids separated by specifically designated amino acid residues. Anchor libraries are therefore subsets of a complete library of a specified length. Anchor libraries can be used to identify essential contacts between a ligand and a target, and have the advantage that only a subset of all possible peptides need be synthesized and screened. In a preferred embodiment, an anchor library is made up of peptides about 16 amino acids long. An anchor library can be prepared by genetic means (*e.g*., by synthesizing a multiplicity of nucleic acid molecules encoding a multiplicity of anchor peptides).

Alternative to forming a peptide library by synthesizing a multiplicity of nucleic acid molecules encoding the peptide library members, a multiplicity of peptides can be synthesized directly by standard chemical methods known in the art. For example, a multiplicity of peptides can be synthesized by "split synthesis" of peptides on solid supports (see, *e.g.*, Lam, K.S. *et al.* (1993) *Bioorg. Med. Chem. Lett.* 3:419-424). Other exemplary chemical syntheses of peptide libraries include the pin method (see, *e.g.*, Geysen, H.M. *et al.* (1984) *Proc. Natl. Acad*. *Sci. USA* 81:3998-4002); the tea-bag method (see, *e.g.,* Houghten, R.A. *et al.* (1985) *Proc. Natl. Acad. Sci. USA* 82:5131-5135); coupling of amino acid mixtures (see, *e.g.*, Tjoeng, F.S. *et al*. (1990) *Int. J. Pept. Protein Res*. 35:141-146; U.S. Patent 5,010,175 to Rutter *et al.*); and synthesis of spatial arrays of compounds (see, *e.g.,* Fodor, S.P.A. *et al.* (1991) *Science* 251:767). Peptide libraries formed by direct synthesis of the peptide library members preferably are bound to a solid support (*e.g.,* a bead or pin, wherein each bead or pin is linked to a single peptide moiety) to facilitate separation of peptides that bind a target from peptides that do not bind a target.

Once the peptide library has been formed. a target of interest is screened with the peptide library to identify one or more library members that bind to the target. Peptides that bind a target can be selected according to known methods, such as biopanning of an immobilized target with a phage display library. In one embodiment, a biotinylated target is immobilized on a streptavidin-coated surface either before or after contacting the target with a peptide library and unbound peptides are removed by washing. Peptide libraries bound to a solid support can be screened by, for example, contacting the peptides immobilized on the solid support with a labeled target and detecting the labeled target bound to library members or, alternatively, by releasing the peptides from the solid support and assaying the resulting solution (see, *e.g.,* Ohlmeyer, M.H.J. *et al.* (1993) *Proc. Natl. Acad. Sci. USA* 90:10922:10926).

Following selection of one or more peptide library members that bind to the target, the amino acid sequence of the peptide is determined according to standard methods. For example, in one embodiment, the amino acid sequence of the peptide is determined by determining the nucleotide sequence of a nucleic acid molecule encoding the peptide and translating the encoded peptide using the genetic code. Nucleotide sequencing can be performed by standard methods (*e.g*., dideoxynucleotide sequencing or Maxam-Gilbert sequencing, either manually or using automated nucleic acid sequencers). Alternatively, in another embodiment, the amino acid sequence of the selected peptide(s) is determined by direct amino acid sequencing of the peptide (*e.g*., by Edman microsequencing, either manually or using automated peptide sequencers).

Once the sequence(s) of the peptide(s) that bind the target selected from the first library has been determined, a peptide motif is generated based on these sequences. As used herein, the term "peptide motif" is intended to include an amino acid consensus sequence that represents preferred amino acid residues within a peptide that are sufficient or essential for binding of the peptide to the target. Typically, the simplest way to generate a peptide motif is to compare the amino acid sequences of all peptides selected from screening a target with the first peptide library and define a peptide motif based on one or more amino acid residues that are conserved within at least two of the selected peptides. If only a single peptide is selected from the initial peptide library screening, the amino acid sequence of this peptide can constitute a peptide motif. Alternatively, when multiple peptides are selected from the initial peptide library screening, the amino acid sequences of each of the selected peptides are optimally aligned and amino acid residues conserved among two or more of the selected peptides can constitute the peptide motif. In addition to, or alternative to, direct alignment and analysis of the primary amino acid sequence of the selected peptides, a peptide motif can be generated by more sophisticated structural analysis of the selected peptides. For example, molecular modelling programs can be employed to determine structural motifs present in the selected peptide(s). Examples of such structural motifs include α-helix, β-turns, and the like (see, *e.g.*, A. Fersht (1985) "Enzyme Structure and Mechanism". 2nd ed., W.H. Freeman and Co., New York). Computer modelling can also be used to calculate properties of active peptides such as hydrophobicity, steric bulk, stacking interactions, dipole moment, and the like. Any of the above-mentioned properties can be included when generating a peptide motif.

In the methods of the invention, after a peptide motif has been generated for a target of interest based on screening of the first library, the target is rescreened with a second, non-peptide library that is designed based on the peptide motif. The second library can be composed of compounds that are designed to have improved properties compared to the peptides selected from screening of the first library, such as increased affinity for the target (*e.g*., predicted by computer modelling of the target with non-peptide compounds designed based on the peptide motif) and/or improved pharmacological properties, such as increased solubility, decreased susceptibility to proteolytic degradation, increased biodistribption and the like. Accordingly, the methods of the invention further comprise the steps of:
forming a second library comprising a multiplicity of non-peptide compounds designed based on the peptide motif;
selecting from the second library at least one non-peptide compound that binds to the target; and
determining the structure or structures of the at least one non-peptide compound that binds to the target.

Preferred non-peptide compounds are those that, although not composed entirely of natural amino acid residues, are nevertheless related structurally to peptides, such as peptidomimetics, peptide derivatives and peptide analogues. As used herein, a "derivative" of a compound X (*e.g*., a peptide) refers to a form of X in which one or more reactive groups on the compound have been derivatized with a substituent group. Examples of peptide derivatives include peptides in which an amino acid side chain, the peptide backbone, or the amino- or carboxy-terminus has been derivatized (*e.g*., peptidic compounds with methylated amide linkages). As used herein an "analogue" of a compound X refers to a compound which retains chemical structures of X necessary for functional activity of X yet which also contains certain chemical structures which differ from X. An example of an analogue of a naturally-occurring peptide is a peptide which includes one or more non-naturally-occurring amino acids. As used herein, a "mimetic" of a compound X refers to a compound in which chemical structures of X necessary for functional activity of X have been replaced with other chemical structures which mimic the conformation of X. Examples of peptidomimetics include peptidic compounds in which the peptide backbone is substituted with one or more benzodiazepine molecules (see *e.g*., James, G.L. *et al*. (1993) *Science* 260:1937-1942) and "retro-inverso" peptides (see U.S. Patent No. 4,522,752 by Sisto), described further below.

The term mimetic, and in particular, peptidomimetic, is intended to include isosteres. The term "isostere" as used herein is intended to include a chemical structure that can be substituted for a second chemical structure because the steric conformation of the first structure fits a binding site specific for the second structure. The term specifically includes peptide back-bone modifications (*i.e.*, amide bond mimetics) well known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[CH₂S], ψ[CH₂NH], ψ[CSNH₂], ψ[NHCO], ψ[COCH₂], and ψ [(E) or (Z) CH=CH]. In the nomenclature used above, ψ indicates the absence of an amide bond. The structure that replaces the amide group is specified within the brackets. Other examples of isosteres include peptides substituted with one or more benzodiazepine molecules (see *e.g*., James, G.L. *et al*. (1993) *Science* 260:1937-1942), peptoids (R.J. Simon *et al*. (1992) *Proc. Natl. Acad. Sci. USA* 89:9367-9371), and the like.

Other possible modifications of peptides include an N-alkyl (or aryl) substitution (ψ[CONR]), backbone crosslinking to construct lactams and other cyclic structures, or retro-inverso amino acid incorporation (ψ[NHCO]). By "inverso" is meant replacing L-amino acids of a sequence with D-amino acids, and by "retro-inverso" or "enantio-retro" is meant reversing the sequence of the amino acids ("retro") and replacing the L-amino acids with D-amino acids. For example, if the parent peptide is Thr-Ala-Tyr, the retro modified form is Tyr-Ala-Thr, the inverso form is thr-ala-tyr, and the retro-inverso form is tyr-ala-thr (lower case letters refer to D-amino acids). Compared to the parent peptide, a retro-inverso peptide has a reversed backbone while retaining substantially the original spatial conformation of the side chains, resulting in a retro-inverso isomer with a topology that closely resembles the parent peptide. See Goodman *et al. "Perspectives in* *Peptide Chemistry"* pp. 283-294 (1981). See also U.S. Patent No. 4,522,752 by Sisto for further description of "retro-inverso" peptides.

Approaches to designing peptide analogues, derivatives and mimetics are known in the art. For example, see Farmer, P.S. in Drug Design (E.J. Ariens, ed.) Academic Press, New York, 1980, vol. 10, pp. 119-143; Ball. J.B. and Alewood, P.F. (1990) *J. Mol. Recognition* 3:55; Morgan, B.A. and Gainor, J.A. (1989) *Ann. Rep. Med. Chem.* 24:243; and Freidinger, R.M. (1989) *Trends Pharmacol. Sci*. 10:270.

The second, non-peptide library can be formed by methods known in the art for combinatorial synthesis of organic compounds. For example, a second library comprising compounds that include modified amino acids (for example, D-amino acids or synthetic amino acids such as phenylglycine) can be synthesized by techniques used for the synthesis of peptide libraries (*e.g*., solid support methods described *supra*). Other organic molecules that have been synthesized on solid supports include benzodiazepines (B.A. Bunin and J.A.A. Ellman (1992) *J*. *Am. Chem. Soc*. 114:10997-10998), peptoids (R.N. Zuckermann *et al*. (1992) *J. Am. Chem. Soc*. 114:10646-10647), peptidyl phosphonates (D.A. Campbell and J.C. Bermak (1994) *J*. *Org. Chem.* 59:658-660), vinylogous polypeptides (M. Hagihara *et al*. (1992) *J. Am. Chem. Soc.* 114:6568-6570), and the like. An alternative synthetic scheme for chemical libraries involves synthesis of compounds on resin beads wherein a coding moiety corresponding to each addition in the synthesis is also coupled to the bead (see *e.g.,* Brenner, S. and Lerner, R.A. (1992) *Proc. Natl. Acad. Sci. USA* 89:5181-5183; Ohlmeyer, M.H.L. *et al*. (1993) *Proc. Natl. Acad. Sci. USA* 90:10922:10926; Still *et al*., PCT publication WO 94/08051). In a preferred embodiment, the second library comprises compounds which include at least one peptide bond (*i.e*., amide bond). In a preferred embodiment, the second library is a library of peptidomimetics.

Preferably, the second library comprises at least about 10² different compounds, more preferably at least 10⁴ different compounds, and still more preferably at least 10⁶ different compounds. Depending upon the size of the non-peptide compounds in the library and the efficiency of synthesis, it may be possible to achieve a second library comprising as many as 10⁸ different compounds or even 10¹⁰ different compounds.

After formation of the second library, the target of interest is screened with the second library, *e.g.*, by the screening methods described above for screening the first library. One or more non-peptide compounds that bind to the target are thereby selected. Preferably, a non-peptide compound selected from the second library that binds to a target has a binding affinity for the target, expressed as an apparent K_{d} (dissociation constant), EC₅₀ (concentration needed for 50% effective binding) or IC₅₀ (concentration needed for 50% inhibition of binding of another compound that binds to the target) of at least about 10⁻⁷ M, more preferably at least about 10⁻⁸ M, and even more preferably at least about 10⁻⁹ M. In a preferred embodiment, compounds identified by screening of the second library have at least 10-fold higher affinity for the target than the peptides identified by screening the first library. More preferably, compounds identified by screening of the second library have at least 100-fold higher affinity for the target than the peptides identified by screening the first library. Even more preferably, compounds identified by screening of the second library have at least 1000-fold higher affinity for the target than the peptides identified by screening the first library.

Following selection of one or more compounds from the second library that bind to the target, the structure of the selected compound(s) is determined. In a preferred embodiment, the structure of the non-peptide compound(s) is determined by the use of a mass spectrometric method. Mass spectrometric methods allow for the rapid, inexpensive, and highly accurate identification of the structure of a compound based on the mass of the compound and on fragments of the compound generated in the mass spectrometer. A preferred mass spectrometric technique is tandem mass spectrometry, sometimes denoted "MS/MS". In tandem mass spectrometry, a sample compound is first ionized and the molecular ion determined. The molecular ion is then cleaved into several smaller fragments, which are then mass-analyzed. The use of mass spectrometry to identify the structure of high-molecular weight compounds, including peptides, has been reported (see, *e.g.,* R.S. Youngquist *et al.* (1995) *J*. *Am. Chem. Soc*. 117:3900; B.J. Egner *et al*. (1995) *J. Org. Chem.* 60:2652-2653). It is believed that tandem mass spectrometry is especially useful for the analysis of non-peptide compounds that contain one or more peptide bonds (*e.g*., peptide derivatives, peptide analogues and/or peptidomimetics) because the peptide bond can be cleaved in the spectrometer to produce fragments that can be analyzed to identify particular subunits of the compound. In certain alternative embodiments, it may be possible to analyze at least a portion of a non-peptide compound by direct amino acid sequencing, *e.g*., by Edman degradation (*e.g*., where the non-peptide compound comprises a peptide portion). Alternatively, in embodiments in which the second library is synthesized in an array (*e.g*., on pins or in an array on a solid surface, *e.g*., a "chip"), the structure of the compound can be determined by the position the compound occupies in the array. In yet other embodiments, in which the second library is an encoded library (*i.e.,* a library in which the structure of the chemical compound has been encoded on a bead, as described in Brenner, S. and Lerner, R.A. (1992) *Proc. Natl. Acad. Sci. USA* 89:5181-5183; Ohlmeyer, M.H.L. *et al*. (1993) *Proc. Natl. Acad. Sci. USA* 90:10922:10926; and Still *et al*., PCT publication WO 94/08051), the structure of the compound can be determined by decoding the encoding moiety.

In a particularly preferred embodiment of the method of the invention, the first (peptide) library is a phage display library, and the non-peptide compound(s) of the second library that bind to the target are analyzed by tandem mass spectrometry. In another particularly preferred embodiment of the methods of the invention, the first (peptide) library is an anchor library, and the compound(s) of the second library that bind to the target are analyzed by tandem mass spectrometry.

The skilled artisan will appreciate that the compound or compounds identified from the second library can be used as a basis for forming further libraries that can be used for further screening of the target. That is, the information gained from the screening of the second library can be used to design another motif, for example a modified peptide motif (e.g. a motif based on the structure of peptide derivatives, peptide analogues and/or peptidomimetics), and a subsequent, third library can be formed comprising compounds designed based on the motif generated from the screening of the second library. The target is then screened with the third library and active compounds identified as previously described herein. This process can be repeated until a compound with a desired binding affinity for the target is obtained.

Another aspect of the invention pertains to a library composed of a multiplicity of non-peptide compounds designed based on a peptide motif, wherein the peptide motif is determined by selecting from a peptide library at least one peptide that binds to a target, determining the sequence or sequences of at least one peptide, preferably multiple peptides, that binds to the target and determining a peptide motif. A library of non-peptide compounds based on a peptide motif can be synthesized by the methods previously described herein. In a preferred embodiment, the non-peptide compounds of the library are peptidomimetics. Additionally or alternatively, the non-peptide compounds can be peptide derivatives and/or peptide analogues. Preferably, the library comprises at least about 10² compounds, more preferably at least about 10⁴ compounds and even more preferably at least about 10⁶ compounds. In one embodiment, the multiplicity of non-peptide compounds are attached to a solid support, such as a plurality of resin beads.

This invention is further illustrated by the following examples which should not be construed as limiting. The contents of all references, patents and published patent applications cited throughout this application are hereby incorporated by reference.

### EXAMPLE 1

In this example, the method of the invention is used to identify one or more compounds that bind to a target that is expressed on the surface of a cell, the luteinizing hormone releasing hormone receptor (LHRH-R), a member of the G-protein coupled, seven transmembrane receptor superfamily.

### Construction of the First Library

A phage anchor library comprising a multiplicity of peptides is used as the first library in the method. The anchor library is comprised of peptides having random amino acid residues distributed throughout domains of alanine (Ala) and/or glycine (Gly) residues. For example, the anchor library can be composed of peptides that are sixteen amino acid residues in length and have the amino acid sequence:

X¹(Ala/Gly)₄X²(Ala/Gly)₄X³(Ala/Gly)₄X⁴

wherein X^{1,} X^{2,} X³ and X⁴ can be any amino acid residue and each can be the same or different from the others.

To prepare the anchor library, a multiplicity of oligonucleotides encoding the peptides are synthesized by standard methods, such as the split synthesis method (See *e.g.,* Cormack and Struhl (1993) *Science* 262:244-248). Synthesis of oligonucleotides for construction of anchor libraries also is described further in U.S. Patent Application Serial No.08/479,660, entitled *Anchor Libraries and Identification of Peptide Binding Sequences,* and corresponding PCT Application No. PCT/US96/09383, the entire contents of both of which are expressly incorporated herein by reference.

Following synthesis, assembled oligonucleotide inserts are cloned into the pfUSE5 phage vector (Smith and Scott (1993) *Methods in Enzymology* 217:228-257), which allows for expression of the encoded peptides as fusions with the pill phage coat protein. The vector (30 µg) is prepared by cleaving with 200 units of endonuclease SfiI in 500 µl of restriction buffer (Buffer #2 from New England BioLabs (NEB), Beverly, MA) for 10 hours. The reaction is terminated with addition of 15 mM EDTA. followed by phenol/chloroform extraction. The vector DNA is recovered by isopropanol precipitation, resuspended in 500 µl of Tris-EDTA (TE) buffer and recovered by ethanol precipitation. The phage vector is ligated to the assembled oligonucleotide inserts at 5 µg/ml vector and three-fold excess assembled insert in ligation buffer (NEB) with 100 units of T4 DNA ligase at 10° C for 16 hours. DNA is purified from the ligation buffer by phenol/chloroform extractions, followed by ethanol precipitations and resuspension in TE buffer.

DNA from the ligation reaction is transformed into electrocompetent MC1061 bacterial host cells (Wertman *et al*. (1986) *Gene* 49:253-262) using 0.5 µg of DNA per 100 µl of cells using 0.2 cm electroporator cells and a BioRad electroporator set at 25 µF, 2.5 KV and 200 ohms. Shocked cells are recovered in SOC media, grown out at 37 °C for 20 minutes and inoculated into LB broth containing 20 µg/ml tetracycline.

Library phage released from the transformed bacterial host cells are isolated after growing the bacterial cells for 16 hours. Phage are separated from cells by centrifugation at 4 °C at 4.2 K rpm for 30 minutes in a Beckman J6 centrifuge, followed by a second centrifugation of the supernatant at 4.2K rpm for 30 minutes. Phage are precipitated with the addition of 150 ml of 16.7 % polyethyleneglycol (PEG)/3.3 M NaCI per liter of supernatant. Mixed solutions are incubated at 4 °C for 16 hours. Precipitated phage are collected by centrifugation at 4.2K rpm in a J6 centrifuge, followed by resuspension in 40 ml of Tris-buffered saline (TBS). Resuspended phage are precipitated again with the addition of 4.5 ml of PEG solution for 4 hours. Phage are collected at 5K rpm in a Beckman JA20 centrifuge at 4° C. Phage are suspended in 7 ml of TBS and brought to 1.3 mg/ml density by the addition of 1 gm of CsCl per 2.226 gm of aqueous solution. Phage are subjected to equilibrium centrifugation in a type 80 rotor at 45K rpm for 40 hours. Phage bands are isolated, diluted 20-fold with TBS and pelleted at 40K rpm in a type 50 rotor. Pellets are resuspended in 0.7 ml of TBS and as is in screening assays, described below, at approximately 3 x 10¹³ phage/ml.

### Screening of the First Library

To identify members of the phage anchor library that bind to LHRH-R, monolayers of cells expressing LHRH-R (such as CHO, COS or SF9 cells transfected to express LHRH-R) adhered to culture dishes are biopanned with the phage library. The phage (in TBS) are incubated with the cells for 1 hour at 4 °C and non-specific phage are removed by washing the cell monolayer with PBS containing 2 % milk or 1 % BSA or 10 % serum for a total of 7 washes over 30 minutes. The remaining phage that are bound to the cells (by way of binding to LHRH-R on the surface of the cells) are recovered by elution with 100 µM glycine, pH 2.2 for 10 minutes. Eluted phage are neutralized with 1 M Tris base.

Eluted phage are amplified by infection of log phase K91 *E. coli* (Lyons and Zinder (1972) *Virology* 49:45-60; Smith and Scott (1993) *Methods in Enzymology* 217:228-257). Approximately 10⁵ phage are amplified by infecting an equal volume of K91 cells with phage at 22 °C for 10 minutes. Infected cells are diluted into 1 ml of LB broth for 30 minutes at 37 °C, followed by an additional dilution with 9 ml of LB containing 20 µg/ml tetracycline and grown overnight. Phage are then separated from cells by centrifugation and purified by PEG precipitation and resuspended at 10¹² phage/ml.

To further enrich for peptides that specifically bind to LHRH-R, amplified phage can be subjected to two additional rounds of biopanning using different cell types expressing LHRH-R in each round of panning and using the binding and amplification conditions described above.

### Generation of a Peptide Motif

After biopanning, individual phage are isolated and sequenced to reveal the DNA sequence that encodes for the displayed peptide in each selected phage. Sequencing is performed by standard methods (*e.g.*, dideoxy sequencing using Sequenase 2.0, United States Biochemical Co., Cleveland OH, according to the manufacturer's protocol).

After obtaining the DNA sequences encoding the selected peptides, the DNA sequences are optimally aligned to generate a peptide motif. The peptide motif is determined from the amino acid residues that are conserved in at least two of the selected peptides. For example, if biopanning of the anchor library leads to selection of four peptides having the following amino acid sequences (standard three-letter abbreviations are used for amino acids): a peptide motif can be generated having the amino acid sequence: (wherein Xaa can be any amino acid residue).

### Construction of a Second Library

Based on the peptide motif generated from screening the target with the first library, a second library comprising a multiplicity of non-peptide compounds is synthesized by standard chemical synthesis methods (see *e.g.*, Youngquist, R.S. *et al*. (1995) *J. Am. Chem. Soc.* 117:3900-3906; Till, J.H. *et al.* (1994) *J. Biol. Chem.* 269:7423-7428; Berman, J. *et al.* (1992) *J. Biol. Chem.* 267:1434-1437). The non-peptide compounds of the library are designed to mimic the peptide motif. For example, to create a non-peptide library based on the peptide motif described above, amino acid derivatives, analogues or mimetics of Ser at position 1, Arg at position 6. Leu at position 11 and/or Xaa at position 16 can be incorporated into the library. Derivatives, analogues and/or mimetics of the repeating Ala/Gly structure can also be incorporated into the library.

One example of a second library synthesized based on the above-described peptide motif is an analog library in which the serine at position 1 of the motif is substituted with homoserine, cyanoalanine, isoglutamine or isoasparagine, the arginine at position 6 of the motif is substituted with citrulline, isopropyllysine, homoarginine, ornithine, homocitrulline, diaminoproprionic acid, aminobenzoic acid or nitroarginine, the leucine at position 11 of the motif is substituted with NorLeu, BuGlycine, cyclohexylalanine, norval, aminobutyrl or various N-methyl aliphatic amino acids and the Xaa at position 16 of the motif is combinatorially derived from the twenty natural amino acids or standard analogs thereof.

Another example of a second library synthesized based on the above-described peptide motif is a library constructed to probe the stereochemical specificity of compounds that bind to the target by alternating D- and L-amino acids in the library. In this case. the library is constructed using the following L-amino acids: Glu, Arg, Asn, Thr, Val, Pro, Met, Tyr and His; and the following D-amino acids: Asp, Lys, Gln, Ser, Cha, Ala, Phe and Trp. The library also contains glycine. This library can define the role of D or L stereochemistry within the selected peptide motif.

Yet another example of a second library synthesized based on the above-described peptide motif is a mimetic library, wherein reduced amide mimetics are incorporated into the compounds of the library via the use of appropriate amino acid aldehyde precursors and the solid phase reductive amination procedure for assembly (Sasaki and Coy (1987) *Peptides* 8:119-120). Mimetics can be incorporated at one site or multiple sites within the library. Appropriate positions include sites within a peptide motif containing an aliphatic or aromatic residue, such as the leucine at position 11 of the above-described peptide motif.

Once synthesized, the library is dissolved in 1-5 % dimethylsulfoxide (DMSO) in water and used in screening assays as described below.

### Screening of the Second Library

To identify members of the second library that bind to LHRH-R, membranes of CHO cells that have been transfected to express LHRH-R on their surface are prepared. One liter quantities of CHO-LHRH-R cells (*e.g*., 10⁹ cells/liter) are grown and harvested. The cells are lysed with a nitrogen bomb (see *e.g.,* Autuori, F. *et al*. (1982) *J. Cell Sci.* 57:1-13). 25 ml of a washed cell suspension (in an isomolar Hanks balanced salt solution/20 mM HEPES buffer, pH 7.4) is placed in a nitrogen bomb at 4 °C with continuous stirring by a magnetic stir bar. and the pressure is adjusted to 4-500 psi, followed by continuous stirring for 20 minutes. Pressure is released into a 50 ml plastic centrifuge tube containing a 100X cocktail of protease inhibitors (0.5 mM PMSF, 10 µg/ml benzamidine, 1 µg/ml leupeptin, final concentrations). The homogenate is centrifuged for 1 hour at 5,000 Xg. The supernatant is subjected to ultracentrifugation at 50.000 Xg for one hour. The final pellet is resuspended to a concentration of about 1 mg/ml and aliquots are frozen in liquid nitrogen until used in binding assays, at which time the aliquots are thawed.

Binding reactions are set up in 12 x 75 mm polypropylene test tubes containing a sample of the second library (final concentration of 10 mg/ml). binding buffer (final concentrations: 10 mM Tris, 0.05 % bovine serum albumin, pH 7.4) and a sample of the cell membrane preparation (approximately 10⁷ cell equivalents per tube) in a total volume of 500 µl. The binding reaction is incubated on ice for 90 minutes. The binding reaction is terminated by fast filtration binding of the mixture using a 12-well cell harvester (Millipore, Milford, MA). Filters (Whatman glass-fiber filters GF/C) are prewashed three times with 300 µl of 10 mM HEPES, 0.01 % sodium azide. 3 ml of HEPES buffer is added to each binding reaction tube and the contents of the tube are poured over the filter in the fast filtration binding apparatus. Two additional aliquots of buffer are added to each tube and poured over the filter. Compounds from the second library that bind to the LHRH-R membrane preparation are retained on the filter, whereas compound that do not bind to the LHRH-R membrane preparation are removed.

### Identification of Compounds that Bind the Target

Compounds from the second library that bind to the LHRH-R membrane preparation are recovered from the filter of the fast filtration binding apparatus. The structures of the selected compounds are determined by tandem mass spectrometry (see *e.g..* Hunt, D.F.. *et al*. (1985) *Anal. Chem*. 57:765-768; Hunt, D.F. *et al*. (1986) *Proc.* *Natl. Acad. Sci. USA* 83:6233-6237; Hunt, D.F. *et al.* (1987) *Proc. Natl. Acad. Sci. USA* 84:620-623; Biemann, K. (1990) *Methods in Enzymology* 193:455-479; Arnott, D. *et al.* (1993) *Clin. Chem.* 39:2005-2010; Metzger, J.W. *et al*. (1994) *Anal. Biochem.* 219:261-277; Brummel, C.L. *et al*. (1994) *Science* 264:399-402).

### EXAMPLE 2

In this example, the method of the invention was used to identify compounds that bind to a fibroblast growth factor (FGF) binding protein, namely an anti-FGF monoclonal antibody. Starting with biologically generated peptide libraries in bacteriophage M13, important amino acids for target binding were defined. Based on the information generated from the bacteriophage library, a combinatorial chemical library with a complexity of approximately 160,000 compounds was designed and synthesized. This combinatorial library contained biased amino acid residues at key positions. A combination of natural amino acids (*i.e*., the 20 amino acids encoded by DNA) and synthetic amino acids (those containing unnatural R-groups, or the D-enantiomer of a natural amino acid) were utilized in the chemical library. Non-peptide compounds that interacted with the target were recovered and were analyzed. Several of these selected non-peptide compounds were synthesized and were shown to be 10-100 fold more potent than the starting natural peptide for target binding. These non-peptide compounds contained unnatural amino acids at 3 or more positions. This example demonstrates that novel, high potency non-peptide compounds containing unnatural amino acids can be discovered using phage display library screening coupled with combinatorial chemistry library screening.

### Derivation of a Peptide Sequence Motif from a Phage Display Library

A monoclonal antibody raised against human basic FGF (bFGF) was used as a biopanning target using a 7-mer peptide library in a bacteriophage M13 vector. To prepare the biopanning plates, four wells of an Immulon4 microtiter plate (Dynatech) were coated with stepavidin (1 µg/well) in 100 mM NaHCO₃, pH 9.5, for 2 hours at room temperature or overnight at 4°C. The strepavidin-coated wells were washed three times with phosphate-buffered saline (PBS). To each well containing strepavidin, biotinylated rat anti mouse antibody κ light chain (PharMingen) was added, at 1 µg/well, in PBS for 30 minutes to 1 hour at room temperature. The wells were then washed again with PBS three times. Non-specific binding sites were blocked with 300 µl/well PBS, 1% dry milk for 1 hour at room temperature. The wells were then washed with PBS, 0.1% dry milk three times, loaded with 50 µl/well PBS, 0.1% dry milk and stored at 4°C.

Monoclonal antibody to bFGF (Sigma) was added to four tubes in 100 µl of PBS, 0.1% dry milk at the following final concentrations: 100 nM, 25 nM, 5 nM and no antibody (control). To each tube containing anti-bFGF antibody, approximately 1x10¹⁰ phage from a seven-mer peptide library were added. The tubes were left at room temperature for 2 hours to allow for specific interaction between the phage and the anti-bFGF antibody.

After the 2 hour room temperature incubation of the phage with the antibody, the PBS, 0.1% milk buffer was removed from the wells of the strepavidin/biotinylated α-mouse complex plates and the corresponding 100 µl of each tube containing the antibody/phage mixture was added to each of the four wells . The solutions were allowed to sit in the wells at room temperature for twenty minutes. To remove unbound or non-specifically bound phage, each well was washed with cold PBS, 0.1% Tween six times, followed by cold PBS washes (six times). Specifically-bound phage were eluted with 100 µl Glycine pH 2.2 for 10 minutes at room temperature. The glycine solution was then removed from wells and added to polypropylene tubes with 6 µl 2M Tris base to neutralize. The phage eluant was titered and the fractional yield determined.

For phage amplification, phage were mixed with concentrated mid log phase *E. coli* strain K91 at room temperature for 5 to 10 minutes. One milliliter of LB was added and the cell were grown at 37°C for 30 min. Nine milliliters of LB^{tet} was then added and the cells were allowed to grow overnight at 37°C. The following morning, the bacteria were pelleted by centrifugation at 5000 rpm for 15 minutes. The supernatant was drained into a fresh 50 ml conical tube and 1.5 ml of PEG/NaCl was added. The tube was chilled at 4°C for 4 hours. The phage were pelleted by centrifugation at 8000 rpm for 30 minutes. The supernatant was drained off and the phage pellet was resuspended in 1 ml PBS. The phage amplification was titered and 1x10¹⁰ phage were used for subsequent rounds of screening as described above.

A number of related phage were selected by this biopanning procedure. The selected peptides. as deduced from DNA sequence analysis of the phage, indicated that the target protein binds to peptides containing the consensus sequence: P-x-G-H-x-K-x (SEQ ID NO: 6). Analysis of the bFGF sequence indicated that the natural epitope for the antibody is P-P-G-H-F-K-D (SEQ ID NO: 7), based on the strong similarity to the peptides selected from the phage display library. Two peptides containing this sequence were synthesized: PPI-416 = G-A-F-P-P-G-H-F-K-D-P-D-R-L (SEQ ID NO: 8) and PPI-432 = P-P-G-H-F-K-D (SEQ ID NO: 7) and tested for their ability to bind to the target. Competitive binding experiments demonstrated that PPI-416 blocked phage containing the sequence P-R-G-H-W-K-Q (SEQ ID NO: 9) from binding to the antibody. Furthermore both PPI-416 and PPI-432 blocked the binding of bFGF to the antibody. Therefore, peptides deduced from phage biopanning bound to the target protein and blocked the interaction of bFGF. The peptide sequence information obtained from phage biopanning was applied to the synthesis of a biased combinatorial peptide library.

### Design and Synthesis of Combinatorial Chemical Libraries

Sequence information derived from phage binding to the target protein, together with the known sequence of bFGF, was used in the design of a secondary biased chemical combinatorial library. Two libraries were synthesized containing a different number of fixed and variable amino acids:

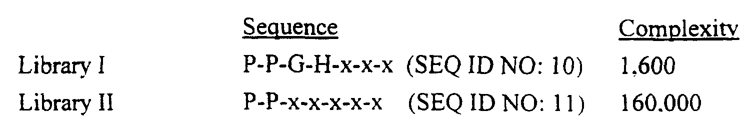

In these libraries, the first 2 or 4 amino acids were fixed to match the amino acids presumed to be involved in target interaction, and the remaining positions were variable. Natural amino acids, L-amino acids with unnatural R-groups, and D-enantiomers of natural amino acids were incorporated into the library. Accordingly, the members of these libraries are referred to herein as "non-peptide compounds". The natural and unnatural amino acids chosen at the variable positions were biased based on the sequences obtained from phage display. For example, many different amino acids were found in the last residue (carboxy-terminal) from the panned phage (position 7). Therefore, 24 different natural and unnatural amino acids were used at this position. Likewise, at the penultimate position (position 6), most phage contained a lysine residue. Therefore, this position was biased in the library synthesis by using predominantly basic natural and unnatural amino acids.

The following abbreviations for unnatural amino acids are used herein:

| Abbreviation | Residue |
|---|---|
| Abu | 2-amino butyric acid |
| Nor-Val | norvaline |
| Hyp | hydroxyproline |
| Cit | citrulline |
| Nal | 3-(2-naphthyl)alanine |
| Orn | ornithine |
| Pal | 3-(3'-pyridyl)alanine |
| Cha | cyclohexylalanine |
| Tic | 1,2,3,4-tetrahydroisoquinoline-3-carboxylic acid |
| pF-Phe | para-fluorophenylalanine |

The composition of the combinatorial library is summarized below in Table 1.

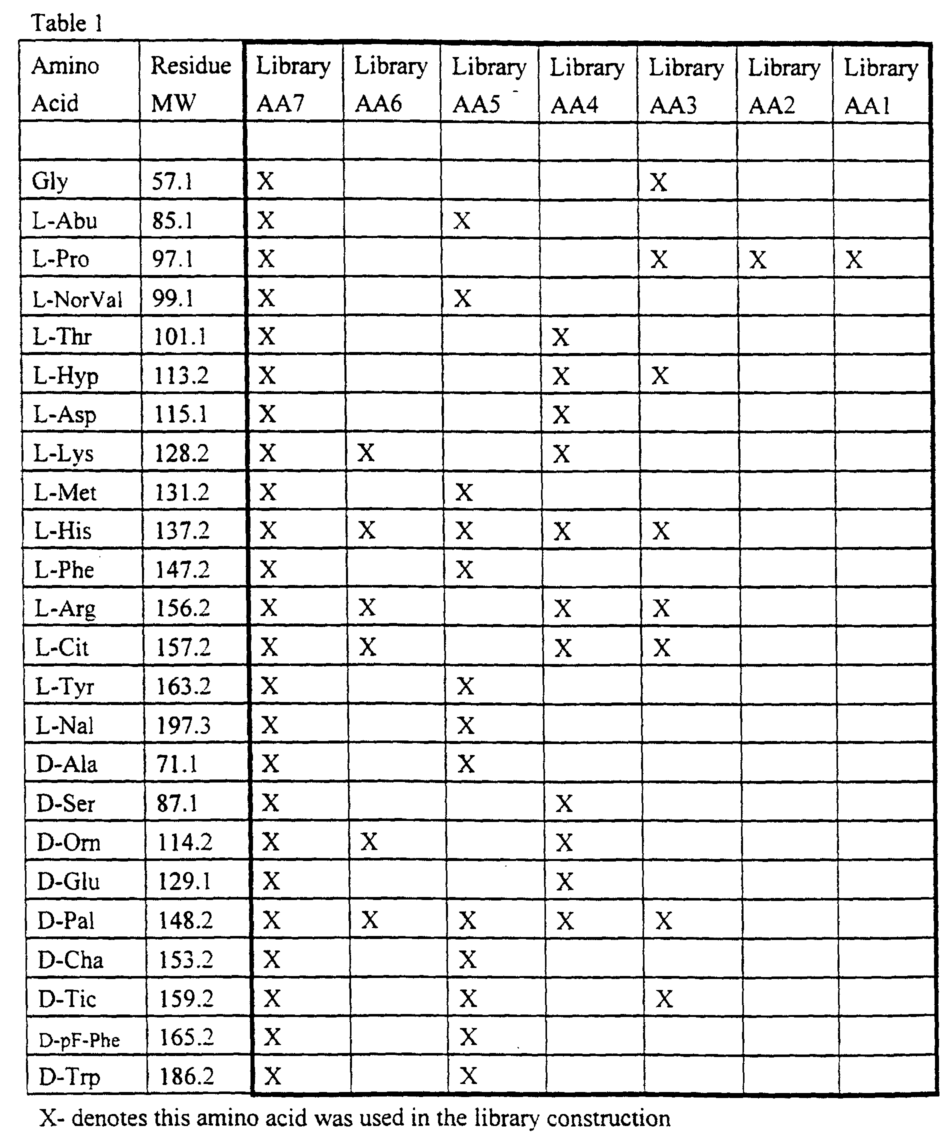

An additional consideration in the design of the library was to use amino acids of different molecular weight at any one position. The sequence of compounds selected from the library ultimately is determined using fragmentation and MS analysis. It is therefore important to choose natural and unnatural amino acids with distinct molecular weights to avoid ambiguity in the sequence determination.

The library was synthesized using equimolar mixtures of each natural or unnatural amino acid at each synthetic step using standard Fmoc chemistry. As a representative example, for Library II (P-P-x-x-x-x-x; SEQ ID NO: 11), the strategy involved use of 1.7 mmoles of a PEG-PAL resin (4.5 g at 0.38 mmol/g), C-terminally modified with an amide, at the start of synthesis. Synthesis using the natural and unnatural amino acids indicated in Table 1 is carried out through five cycles (*i.e.,* amino acid positions 7-3 as indicated in Table 1). At this point. 0.1 mmole of the resin is removed (1/17 of total) and the synthesis is completed with Pro for position 2 (AA2 as indicated in Table 1), Pro for position 1 (AA 1 as indicated in Table 1) and an N-terminal acetylation to prepare a test library of approximately 2 x 10⁵ complexity. For coupling, depending on the diversity of each step, individual amino acids are weighed to provide a total of 3.4 mmoles per cycle. The combined amino acids are dissolved in 20 ml of NMP so that 10 ml (equivalent) is used for the first coupling and 10 ml for the second coupling. The first coupling uses HBTU/HOBT chemistry and the second coupling uses DCC/HOAt coupling strategy. Limiting amounts of Fmoc amino acids are used at each cycle to achieve uniform distribution of each natural or unnatural amino acid. Coupling efficiency is checked with ninhydrin reagent after each cycle of synthesis to assure the reactions went to completion. Following synthesis, the library is cleaved from the resin, precipitated and lyophilized. To remove scavengers, a chromatography step is performed following lyophilization. The final library is evaluated by HPLC to assure the complexity of the library.

### Library Characterization

Each complete library, containing a mixture of approximately 1600 non-peptide compounds (Library I) or 160,000 non-peptide compounds (Library II), was analyzed by MS. The mass spectrum of each library was consistent with the distribution of molecular weights predicted from the diverse amino acids used for the synthesis.

The activities of the libraries were tested using an FGF binding assay. In comparison to the peptide PPI-432 (P-P-G-H-F-K-D; SEQ ID NO: 7), the unselected Library I was approximately 10-fold less potent in blocking the binding of FGF to the target protein. Similarly, the unselected Library II was about 1000-fold less potent that PPI-432. These data are consistent with the libraries containing a mixture of diverse non-peptide compounds whose average potency is decreased relative to the original peptide upon which the library was designed. Since Libraries I and II are highly diverse, the active non-peptide compounds should have a spectrum of potencies which cumulatively yield the average potency seen in the library. Selective enrichment of non-peptide compounds that interact with the target should be proportional to their binding affinities, allowing recovery of the most active components in the library.

### Selection of Non-Peptide Compounds for Functional Analysis

The target FGF binding protein (α-FGF-mAb) was biotinylated using activated N-hydroxy-succinimide ester. The non-peptide compound library (500 µg) was incubated with the biotinylated target antibody (17.5 µg) in 500 µl phosphate buffered saline (PBS) at 4°C for 2 hours. Bound non-peptide compounds were recovered in complex with the antibody by capture on magnetic streptavidin beads as follows.

Magnetic streptavidin beads (100 µl beads per reaction) were immobilized on a magnet and washed twice with PBS. The beads were then resuspended in 100 µl PBS per sample. Washed beads (100 µl) were added to each antibody-library binding reaction and the mixture was incubated for 15 minutes at 4° C with constant rotation. The tubes were spun briefly to clear beads off the wall of the tube and the beads were then washed three times with PBS. The beads were then pelleted by centrifugation or by use of a magnet and the PBS removed. To elute bound non-peptide compounds, 100 µl of 10% acetic acid was added and the mixture was incubated at 4° C for 15 minutes. The beads were immobilized on the magnet and the supernatant, containing the eluted non-peptide compounds, was recovered.

The eluted-non-peptide compounds were brought to dryness under vacuum. Recovered non-peptide compounds were dissolved in 50 µl water and tested for their ability to block the interaction of bFGF with the target protein. (Assuming 100% recovery of non-peptide compounds and 100% binding to bivalent antibody, the amount of recovered non-peptide compounds was calculated to be 200 ng). Binding experiments demonstrated that relative to the unselected library, the potency of the selected population of non-peptide compounds was increased by approximately 1000-fold.

### Selection of Non-Peptide Compounds for MS and Sequence Analysis

Selection of peptide for liquid chromatography/mass spectrometry (LC/MS) analysis and sequence analysis was performed as described above, except 50 µg of antibody and 1 mg of library was used. Selected non-peptide compounds were eluted with acetic acid and dried by lyophilization.

The selected non-peptide compounds were first analyzed by coupled LC/MS. The results showed the presence of non-peptide compounds with various chromatographic elution times. Individual fractions across the chromatographic gradient were next analyzed by MS. Different fractions contained a relatively small number of non-peptide compounds with distinct molecular masses. For example, chromatographic fraction 205-215 contained a major M/Z ion of 485.7. This doubly charged ion corresponds to a non-peptide compound with a molecular mass of approximately 971. This peak was fragmented and analyzed by tandem MS in order to deduce the structure of the non-peptide compound. The fragmentation pattern of this peptide together with the known natural and non-natural amino acids used in the library synthesis allowed for the unambiguous determination of the structure of the non-peptide compound, which structure is as follows: Pro-Pro-Gly-His-Nal-Lys-Nal (SEQ ID NO: 12). In a similar manner, the structures of several other major MS peaks were determined and are summarized below in Table 2:

**Table 2:**

| Structure of Compounds that Bind to Anti-FGF Monoclonal Antibody | | | | | | | | |
|---|---|---|---|---|---|---|---|---|
| | 1 | 2 | 3 | 4 | 5 | 6 | 7 | SEQ ID NO: |
| (PPI-432) | Pro | Pro | Gly | His | Phe | Lys | Asp | 7 |
| (PPI-652) | Pro | Pro | Gly | His | Nal | Lys | Nal | 12 |
| (PPI-654) | Pro | Pro | Gly | His | Nal | Lys | D-pF-Phe | 13 |
| | Pro | Pro | Gly | His | Phe | Lys | Nal | 14 |
| | Pro | Pro | Gly | His | Nal | Lys | Abu | 15 |
| | Pro | Pro | Gly | His | Nal | Lys | D-Ala | 16 |
| | Pro | Pro | Pal | x | x | x | x | 17 |

### Activity of Non-Peptide Compounds Selected from the Secondary Combinatorial Chemical Library

Several of the non-peptide compounds selected from the combinatorial library were made synthetically and compared to the starting peptide (PPI-432) for their ability to bind to the target protein. The compounds were synthesized with an amino-terminal acetyl group and a carboxy-terminal amide. The potency of two non-peptide compounds (PPI-652 and PPI-654, shown in Table 2), containing modified amino acids at positions 5 and 7, were compared to the starting peptide PPI-432 in their ability to inhibit radiolabeled FGF binding to biotinylated anti-FGF antibody. The results of this experiment are shown in Figure 1, which demonstrate that PPI-652 and PPI-654 exhibit 10-100-fold higher binding affinity for FGF than the starting peptide PPI-432. Thus, the recovery of non-peptide compounds from diverse combinatorial libraries following selection against target correlates with the potency of those non-peptide compounds in target binding.

## Claims

1. A method for identifying a compound that binds to a target, the method comprising:
a) forming a first library comprising a multiplicity of peptides, wherein said first library is biologically generated;
b) selecting from the first library at least one peptide that binds to the target;
c) determining the sequence or sequences of the at least one peptide that binds to the target, thereby generating a peptide motif;
d) forming a second library comprising a multiplicity of non-natural amino acid containing compounds designed based on the peptide motif;
e) selecting from the second library at least one non-natural amino acid containing compound that binds to the target; and
f) determining the structure or structures of the at least one non-natural amino acid containing compound that binds to the target;
thereby identifying a compound that binds to the target.

2. The method of claim 1, wherein said biologically generated library is an external library.

3. The method of claim 2, wherein said external library is a phage display library.

4. The method of claim 1, wherein said biologically generated library is an internal library.

5. A method for identifying a compound that binds to a target, the method comprising:
a) forming a first library comprising an anchor library of a multiplicity of peptides;
b) selecting from the first library at least one peptide that binds to the target;
c) determining the sequence or sequences of the at least one peptide that binds to the target, thereby generating a peptide motif;
d) forming a second library comprising a multiplicity of non-natural amino acid containing compounds designed based on the peptide motif;
e) selecting from the second library at least one non-natural amino acid containing compound that binds to the target; and
f) determining the structure or structures of the at least one non-natural amino acid containing compound that binds to the target;
thereby identifying a compound that binds to the target.

6. The method of claim 1 or 5, wherein the first library comprises at least about 10⁶ peptides.

7. The method of claim 1 or 5, wherein the first library comprises at least about 10⁹ peptides.

8. The method of claim 1 or 5, wherein the first library comprises at least about 10¹² peptides.

9. The method of claim 1 or 5, wherein step c) comprises determining the nucleotide sequence of a nucleic acid molecule or molecules that encode the at least one peptide.

10. The method of claim 1 or 5, wherein step c) comprises determining the amino acid sequence or sequences of the at least one peptide.

11. The method of claim 1 or 5, wherein the second library comprises at least one peptide derivative.

12. The method of claim 1 or 5, wherein the second library comprises at least one peptide analogue.

13. The method of claim 1 or 5, wherein the second library comprises at least one peptidomimetic.

14. The method of claim 1 or 5, wherein the second library comprises at least about 10² non-natural amino acid containing compounds.

15. The method of claim 1 or 5, wherein the second library comprises at least about 10⁴ non-natural amino acid containing compounds.

16. The method of claim 1 or 5, wherein the second library comprises at least about 10⁶ non-natural ammo acid containing compounds.

17. The method of claim 1 or 5, wherein step f) comprises analyzing the at least one non-natural amino acid containing compound by a mass spectrometric method.

18. The method of claim 17, wherein the mass spectrometric method comprises tandem mass spectrometry.

19. The method of claim 1 or 5, wherein the compound that binds to a target has a binding affinity for the target, expressed as an apparent K_{d}, EC₅₀ or IC₅₀, of at least about 10⁻⁷ M.

20. The method of claim 1 or 5, wherein the compound that binds to a target has a binding affinity for the target, expressed as an apparent K_{d}, EC₅₀ or IC₅₀, of at least about 10⁻⁸ M.

21. The method of claim 1 or 5, wherein the compound that binds to a target has a binding affinity for the target, expressed as an apparent K_{d}, EC₅₀ or IC₅₀, of at least about 10⁻⁹ M.

22. The method of claim 1 or 5, wherein the at least one non-natural amino acid containing compound that binds to the target as selected in step e) has at least a 10-fold higher affinity for the target than the at least one peptide that binds the target as selected in step b).

23. The method of claim 1 or 5, wherein the at least one non-natural amino acid containing compound that binds to the target as selected in step e) has at least a 100-fold higher affinity for the target than the at least one peptide that binds the target as selected in step b).

24. The method of claim 1 or 5, wherein the at least one non-natural amino acid containing compound that binds to the target as selected in step e) has at least a 1000-fold higher affinity for the target than the at least one peptide that binds the target as selected in step b).

25. The method of claim 1, further comprising:
g) forming a third library comprising a multiplicity of non-natural amino acid containing compounds designed based on the structure or structures of the non-natural amino acid containing compound or compounds determined in step f);
h) selecting from the third library at least one non-natural amino acid containing compound that binds to the target; and
i) determining the structure or structures of the at least one non-natural amino acid containing compound selected in step H);
thereby identifying a compound that binds to the target.

26. A library composed of a multiplicity of non-natural amino acids designed based on a peptide motif, wherein the peptide motif is determined by selecting from a peptide library at least one peptide that binds to a target, determining the sequence or sequences of the at least one peptide that binds to the target and determining a peptide motif.

27. The library of claim 26, wherein the library comprises at least one peptidomimetic.

28. The library of claim 26, wherein the library comprises at least about 10² non-natural amino acid containing compounds.

29. The library of claim 26, wherein the library comprises at least about 10⁴ non-natural amino acid containing compounds.

30. The library of claim 26, wherein the library comprises at least about 10⁶ non-natural amino acid containing compounds.

31. The library of claim 26, wherein the multiplicity of non-natural amino acid containing compounds are attached to a solid support.

## Patentansprüche

1. Verfahren zum Identifizieren einer Verbindung, die an ein Target bindet, wobei das Verfahren Folgendes umfasst:
a) Ausbilden einer ersten Bibliothek, die eine Vielzahl von Peptiden aufweist, wobei die erste Bibliothek biologisch erzeugt ist;
b) Auswählen zumindest eines Peptids aus der ersten Bibliothek, das an das Target bindet;
c) Bestimmen der Sequenz oder der Sequenzen des zumindest einen Peptids, das an das Target bindet, wodurch ein Peptidmotiv erzeugt wird;
d) Ausbilden einer zweiten Bibliothek, die eine Vielzahl von nicht-natürliche Aminosäure enthaltenden Verbindungen umfasst, die auf Grundlage des Peptidmotivs entwickelt wurden;
e) Auswählen zumindest einer nicht-natürliche Aminosäure enthaltenden Verbindung, die an das Target bindet, aus der zweiten Bibliothek; und
f) Bestimmen der Struktur oder der Strukturen der zumindest einen nicht-natürliche Aminosäure enthaltenden Verbindung, die an das Target bindet;
wodurch eine Verbindung identifiziert wird, die an das Target bindet.

2. Verfahren nach Anspruch 1,
wobei die biologisch erzeugte Bibliothek eine externe Bibliothek ist.

3. Verfahren nach Anspruch 2,
wobei die externe Bibliothek eine Phagen-Display-Bibliothek ist.

4. Verfahren nach Anspruch 1,
wobei die biologisch erzeugte Bibliothek eine interne Bibliothek ist.

5. Verfahren zum Identifizieren einer Verbindung, die an ein Target bindet, wobei das Verfahren Folgendes umfasst:
a) Ausbilden einer ersten Bibliothek, die eine Ankerbibliothek einer Vielzahl von Peptiden umfasst;
b) Auswählen zumindest eines Peptids aus der ersten Bibliothek, das an das Target bindet;
c) Bestimmen der Sequenz oder der Sequenzen des zumindest einen Peptids, das an das Target bindet, wodurch ein Peptidmotiv erzeugt wird;
d) Ausbilden einer zweiten Bibliothek, die eine Vielzahl von nicht-natürliche Aminosäure enthaltenden Verbindungen umfasst, die auf Grundlage des Peptidmotivs entwickelt wurden;
e) Auswählen zumindest einer nicht-natürliche Aminosäure enthaltenden Verbindung, die an das Target bindet, aus der zweiten Bibliothek; und
f) Bestimmen der Struktur oder der Strukturen der zumindest einen nicht-natürliche Aminosäure enthaltenden Verbindung, die an das Target bindet;
wodurch eine Verbindung identifiziert wird, die an das Target bindet.

6. Verfahren nach Anspruch 1 oder 5,
wobei die erste Bibliothek zumindest ungefähr 10⁶ Peptide umfasst.

7. Verfahren nach Anspruch 1 oder 5,
wobei die erste Bibliothek zumindest ungefähr 10⁹ Peptide umfasst.

8. Verfahren nach Anspruch 1 oder 5,
wobei die erste Bibliothek zumindest ungefähr 10¹² Peptide umfasst.

9. Verfahren nach Anspruch 1 oder 5,
wobei Schritt c) eine Bestimmung der Nucleotidsequenz eines Nucleinsäuremoleküls oder - moleküle umfasst, das (die) das zumindest eine Peptid kodiert (kodieren).

10. Verfahren nach Anspruch 1 oder 5,
wobei Schritt c) eine Bestimmung der Aminosäuresequenz oder -sequenzen des zumindest einen Peptids umfasst.

11. Verfahren nach Anspruch 1 oder 5,
wobei die zweite Bibliothek zumindest ein Peptidderivat umfasst.

12. Verfahren nach Anspruch 1 oder 5,
wobei die zweite Bibliothek zumindest ein Peptidanalogon umfasst.

13. Verfahren nach Anspruch 1 oder 5,
wobei die zweite Bibliothek zumindest ein Peptidomimetikum umfasst.

14. Verfahren nach Anspruch 1 oder 5,
wobei die zweite Bibliothek zumindest ungefähr 10² nicht-natürliche Aminosäure enthaltende Verbindungen umfasst.

15. Verfahren nach Anspruch 1 oder 5,
wobei die zweite Bibliothek zumindest ungefähr 10⁴ nicht-natürliche Aminosäure enthaltende Verbindungen umfasst.

16. Verfahren nach Anspruch 1 oder 5,
wobei die zweite Bibliothek zumindest ungefähr 10⁶ nicht-natürliche Aminosäure-enthaltende Verbindungen umfasst.

17. Verfahren nach Anspruch 1 oder 5,
wobei Schritt f) eine Untersuchung der zumindest einen nicht-natürliche Aminosäure enthaltenden Verbindung durch ein massenspektrometisches Verfahren umfasst.

18. Verfahren nach Anspruch 17,
wobei das massenspektrometrische Verfahren eine Tandem-Massenspektrometrie umfasst.

19. Verfahren nach Anspruch 1 oder 5,
wobei die Verbindung, die an ein Target bindet, eine Bindungsaffinität für das Target aufweist, ausgedrückt als ein apparenter K_{d}, EC₅₀ oder IC₅0, von zumindest ungefähr 10⁻⁷ M.

20. Verfahren nach Anspruch 1 oder 5,
wobei die Verbindung, die an ein Target bindet, eine Bindungsaffinität für das Target, ausgedrückt als ein apparenter K_{d}, EC₅₀ oder IC₅₀, von zumindest ungefähr 10⁻⁸ M aufweist.

21. Verfahren nach Anspruch 1 oder 5,
wobei die Verbindung, die an ein Target bindet, eine Bindungsaffinität für das Target, ausgedrückt als ein apparenter K_{d}, EC₅₀ oder IC₅₀, von zumindest ungefähr 10⁻⁹ M aufweist.

22. Verfahren nach Anspruch 1 oder 5,
wobei die zumindest eine nicht-natürliche Aminosäure enthaltende, wie in Schritt e) ausgewählte, Verbindung, die an das Target bindet, eine zumindest zehnfach höhere Affinität für das Target als das zumindest eine wie in Schritt b) ausgewählte Peptid aufweist, das das Target bindet.

23. Verfahren nach Anspruch 1 oder 5,
wobei die zumindest eine nicht-natürliche Aminosäure enthaltende, wie in Schritt e) ausgewählte Verbindung, die an das Target bindet, eine zumindest hundertfach höhere Affinität für das Target als das zumindest eine wie in Schritt b) ausgewählte Peptid aufweist, das das Target bindet.

24. Verfahren nach Anspruch 1 oder 5,
wobei die zumindest eine nicht-natürliche Aminosäure enthaltende, wie in Schritt e) ausgewählte, Verbindung, die an das Target bindet, eine zumindest tausendfach höhere Affinität für das Target als das zumindest eine, wie in Schritt b) ausgewählte, Peptid aufweist, das das Target bindet.

25. Verfahren nach Anspruch 1,
das weiterhin Folgendes umfasst:
g) Ausbilden einer dritten Bibliothek, die eine Vielzahl von nicht-natürliche Aminosäure enthaltenden Verbindungen umfasst, entwickelt auf Grundlage der Struktur oder Strukturen der nicht-natürliche Aminosäure enthaltenden Verbindung oder Verbindungen, die in Schritt f) bestimmt wurden;
h) Auswählen zumindest einer nicht-natürliche Aminosäure enthaltenden Verbindung aus der dritten Bibliothek, die an das Target bindet; und
i) Bestimmen der Struktur oder der Strukturen der zumindest einen nicht-natürliche Aminosäure enthaltenden Verbindung, die in Schritt h) ausgewählt wurde; wodurch eine Verbindung identifiziert wird, die an das Target bindet.

26. Bibliothek, die aus einer Vielzahl von nicht-natürliche Aminosäure enthaltenden Verbindungen zusammengesetzt ist, die auf Grundlage eines Peptidmotivs entwickelt wurden, wobei das Peptidmotiv durch Auswählen zumindest eines Peptids, das an ein Target bindet, aus einer Peptidbibliothek, durch Bestimmen der Sequenz oder der Sequenzen des zumindest einen Peptids, das an das Target bindet, und durch Bestimmen eines Peptidmotivs, bestimmt wird.

27. Bibliothek nach Anspruch 26,
wobei die Bibliothek zumindest ein Peptidomimetikum umfasst.

28. Bibliothek nach Anspruch 26,
wobei die Bibliothek zumindest ungefähr 10² nicht-natürliche Aminosäure enthaltende Verbindungen aufweist.

29. Bibliothek nach Anspruch 26,
wobei die Bibliothek zumindest ungefähr 10⁴ nicht-natürliche Aminosäure enthaltende Verbindungen umfasst.

30. Bibliothek nach Anspruch 26,
wobei die Bibliothek zumindest ungefähr 10⁶ nicht-natürliche Aminosäure enthaltende Verbindungen umfasst.

31. Bibliothek nach Anspruch 26,
wobei die Vielzahl von nicht-natürliche Aminosäure enthaltenden Verbindungen an einem festen Träger befestigt ist.

## Revendications

1. Procédé pour identifier un composé qui se lie à une cible, le procédé comprenant le fait de :
a) former une première bibliothèque comprenant une multitude de peptides ;
b) sélectionner, à partir de la première bibliothèque, au moins un peptide qui se lie à la cible;
c) déterminer la séquence ou les séquences d'au moins un peptide qui se lie à la cible, pour ainsi générer un motif peptidique ;
d) former une deuxième bibliothèque comprenant une multitude de composés contenant des acides aminés non naturels, conçus en se basant sur le motif peptidique ;
e) sélectionner, à partir de la deuxième bibliothèque, au moins un composé non naturel contenant des acides aminés, qui se lie à la cible ; et
f) déterminer la structure ou les structures dudit ou desdits composés contenant des acides aminés non naturels, qui se lient à la cible ;
pour ainsi identifier un composé qui se lie à la cible.

2. Procédé selon la revendication 1, dans lequel ladite bibliothèque générée par voie biologique est une bibliothèque externe.

3. Procédé selon la revendication 2, dans lequel ladite bibliothèque externe est une bibliothèque de visualisation de phages.

4. Procédé selon la revendication 1, dans lequel ladite bibliothèque générée par voie biologique est une bibliothèque interne.

5. Procédé pour identifier un composé qui se lie à une cible, le procédé comprenant le fait de :
a) former une première bibliothèque comprenant une bibliothèque d'ancrage d'une multitude de peptides ;
b) sélectionner, à partir de la première bibliothèque au moins un peptide qui se lie à la cible ;
c) déterminer la séquence ou les séquences d'au moins un peptide qui se lie à la cible, pour ainsi générer un motif peptidique ;
d) former une deuxième bibliothèque comprenant une multitude de composés contenant des acides aminés non naturels, conçus en se basant sur le motif peptidique ;
e) sélectionner, à partir de la deuxième bibliothèque, au moins un composé non naturel contenant des acides aminés, qui se lie à la cible ; et
f) déterminer la structure ou les structures dudit ou desdits composés contenant des acides aminés non naturels, qui se lient à la cible ;
pour ainsi identifier un composé qui se lie à la cible.

6. Procédé selon la revendication 1 ou 5, dans lequel la première bibliothèque comprend au moins environ 10⁶ peptides.

7. Procédé selon la revendication 1 ou 5, dans lequel la première bibliothèque comprend au moins environ 10⁹ peptides.

8. Procédé selon la revendication 1 ou 5, dans lequel la première bibliothèque comprend au moins environ 10¹² peptides.

9. Procédé selon la revendication 1 ou 5, dans lequel l'étape c) comprend le fait de déterminer la séquence nucléotidique d'une molécule ou de molécules d'acides nucléiques qui encodent ledit ou lesdits peptides.

10. Procédé selon la revendication 1 ou 5, dans lequel l'étape c) comprend le fait de déterminer la séquence ou les séquences d'acides aminés dudit ou desdits peptides.

11. Procédé selon la revendication 1 ou 5, dans lequel la deuxième bibliothèque comprend au moins un dérivé de peptide.

12. Procédé selon la revendication 1 ou 5, dans lequel la deuxième bibliothèque comprend au moins un analogue de peptide.

13. Procédé selon la revendication 1 ou 5, dans lequel la deuxième bibliothèque comprend au moins une substance mimétique de peptide.

14. Procédé selon la revendication 1 ou 5, dans lequel la deuxième bibliothèque comprend au moins environ 10² composés contenant des acides aminés non naturels.

15. Procédé selon la revendication 1 ou 5, dans lequel la deuxième bibliothèque comprend au moins environ 10⁴ composés contenant des acides aminés non naturels.

16. Procédé selon la revendication 1 ou 5, dans lequel la deuxième bibliothèque comprend au moins environ 10⁶ composés contenant des acides aminés non naturels.

17. Procédé selon la revendication 1 ou 5, dans lequel l'étape f) comprend le fait d'analyser ledit ou desdits composés contenant des acides aminés non naturels via un procédé de spectrométrie de masse.

18. Procédé selon la revendication 17, dans lequel le procédé de spectrométrie de masse comprend une spectrométrie de masse en tandem.

19. Procédé selon la revendication 1 ou 5, dans lequel le composé qui se lie à une cible possède une affinité de liaison pour la cible, exprimée sous la forme d'une valeur K_{d} apparente, de la valeur CE₅₀ ou de la valeur CI₅₀, d'au moins environ 10⁻⁷ M.

20. Procédé selon la revendication 1 ou 5, dans lequel le composé qui se lie à une cible possède une affinité de liaison pour la cible, exprimée sous la forme d'une valeur K_{d} apparente, de la valeur CE₅₀ ou de la valeur CI₅₀, d'au moins environ 10⁻⁸ M.

21. Procédé selon la revendication 1 ou 5, dans lequel le composé qui se lie à une cible possède une affinité de liaison pour la cible, exprimée sous la forme d'une valeur K_{d} apparente, de la valeur CE₅₀ ou de la valeur CI₅₀, d'au moins environ 10⁻⁹ M.

22. Procédé selon la revendication 1 ou 5, dans lequel le composé contenant des acides aminés non naturels, qui se lie à la cible, tel que sélectionné à l'étape e), possède une affinité pour la cible supérieure à concurrence d'au moins 10 fois à celle dudit ou desdits peptides qui se lient à la cible, tels que sélectionnés à l'étape b).

23. Procédé selon la revendication 1 ou 5, dans lequel le composé contenant des acides aminés non naturels, qui se lie à la cible, tel que sélectionné à l'étape e), possède une affinité pour la cible supérieure à concurrence d'au moins 100 fois à celle dudit ou desdits peptides qui se lient à la cible, tels que sélectionnés à l'étape b).

24. Procédé selon la revendication 1 ou 5, dans lequel le composé contenant des acides aminés non naturels, qui se lie à la cible, tel que sélectionné à l'étape e), possède une affinité pour la cible supérieure à concurrence d'au moins 1000 fois à celle dudit ou desdits peptides qui se lient à la cible, tels que sélectionnés à l'étape b).

25. Procédé selon la revendication 1, comprenant en outre le fait de :
g) former une troisième bibliothèque comprenant une multitude de composés contenant des acides aminés non naturels conçus en se basant sur la structure ou sur les structures du composé ou des composés contenant des acides aminés non naturels, déterminés à l'étape f) ;
h) sélectionner, à partir de la troisième bibliothèque, au moins un composé contenant des acides aminés non naturels, qui se lie à la cible ; et
i) déterminer la structure ou les structures dudit ou desdits composés contenant des acides aminés non naturels, sélectionnés à l'étape h) ;
pour ainsi identifier un composé qui se lie à la cible.

26. Bibliothèque composée d'une multitude de composés contenant des acides aminés non naturels conçus en se basant sur un motif peptidique, dans laquelle le motif peptidique est déterminé en sélectionnant, à partir d'une bibliothèque de peptides, au moins un peptide qui se lie à une cible, en déterminant la séquence ou les séquences dudit ou desdits peptides qui se lient à la cible et en déterminant un motif peptidique.

27. Bibliothèque selon la revendication 26, dans laquelle la bibliothèque comprend au moins une substance mimétique d'un peptide.

28. Bibliothèque selon la revendication 26, dans laquelle la bibliothèque comprend au moins environ 10² composés contenant des acides aminés non naturels.

29. Bibliothèque selon la revendication 26, dans laquelle la bibliothèque comprend au moins environ 10⁴ composés contenant des acides aminés non naturels.

30. Bibliothèque selon la revendication 26, dans laquelle la bibliothèque comprend au moins environ 10⁶ composés contenant des acides aminés non naturels.

31. Bibliothèque selon la revendication 26, dans laquelle la multitude de composés contenant des acides aminés non naturels sont fixés sur un support solide.


### SEQUENCE LISTING

(1) GENERAL INFORMATION:
   (i) APPLICANT:
      (A) NAME: PRAECIS PHARMACEUTICALS INCORPORATED
      (B) STREET: ONE HAMPSHIRE STREET
      (C) CITY: CAMBRIDGE
      (D) STATE: MASSACHUSETTS
      (E) COUNTRY: USA
      (F) POSTAL CODE (ZIP): 02139-1572
   (ii) TITLE OF INVENTION: Methods for Identifying Compounds that Bind to a Target
   (iii) NUMBER OF SEQUENCES: 17
   (iv) CORRESPONDENCE ADDRESS:
      (A) ADDRESSEE: LAHIVE & COCKFIELD
      (B) STREET: 60 State Street, suite 510
      (C) CITY: Boston
      (D) STATE: Massachusetts
      (E) COUNTRY: USA
      (F) ZIP: 02109-1875
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE:
      (C) CLASSIFICATION:
   (vii) PRIOR APPLICATION DATA:
      (A) APPLICATION NUMBER: US 08/573,786
      (B) FILING DATE: 18-DEC-1995
   (viii) ATTORNEY/AGENT INFORMATION:
      (A) NAME: DeConti. Giulio A., Jr.
      (B) REGISTRATION NUMBER: 31,503
      (C) REFERENCE/DOCKET NUMBER: PPI-012CPPC
   (ix) TELECOMMUNICATION INFORMATION:
      (A) TELEPHONE: (617)227-7400
      (B) TELEFAX: (617)227-5941
(2) INFORMATION FOR SEQ ID NO:1:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 2-5,7-10,12-15
      (D) OTHER INFORMATION: /note= Xaa is Ala or Gly
   (xi) SEQUENCE DESCRIPTION: SEQ ID NO:1:
   Ser Xaa Xaa Xaa Xaa Arg Xaa Xaa Xaa Xaa Leu Xaa Xaa Xaa Xaa Met
   1               5                   10                  15
(2) INFORMATION FOR SEQ ID NO:2:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION:. 2-5,7-10,12-15
      (D) OTHER INFORMATION: /note= Xaa is Ala or Gly
   (xi) SEQUENCE DESCRIPTION: SEQ ID NO:2:
   Ser Xaa Xaa Xaa Xaa Lys Xaa Xaa Xaa Xaa Leu Xaa Xaa Xaa Xaa Gln
   1               5                   10                  15
(2) INFORMATION FOR SEQ ID NO:3:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 2-5,7-10,12-15
      (D) OTHER INFORMATION: /note= Xaa is Ala or Gly
   (xi) SEQUENCE DESCRIPTION: SEQ ID NO:3:
   Phe Xaa Xaa Xaa Xaa Arg Xaa Xaa Xaa Xaa Leu Xaa Xaa Xaa Xaa Thr
   1               5                   10                  15
(2) INFORMATION FOR SEQ ID NO:4:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 2-5,7-10,12-15
      (D) OTHER INFORMATION: /note= Xaa is Ala or Gly
   (xi) SEQUENCE DESCRIPTION: SEQ ID NO:4:
   Ser Xaa Xaa Xaa Xaa Asn Xaa Xaa Xaa Xaa Leu Xaa Xaa Xaa Xaa Ile
   1               5                   10                  15
(2) INFORMATION FOR SEQ ID NO:5:
   (i) SEQUENCE CHARACTERISTICS:
      (A) LENGTH: 16 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 2-5,7-10,12-15
      (D) OTHER INFORMATION: /note= Xaa is Ala or Gly
   (ix) FEATURE:
      (A) NAME/KEY: Modified-site
      (B) LOCATION: 16
      (D) OTHER INFORMATION: /note= Xaa is any amino acid
      (xi) SEQUENCE DESCRIPTION: SEQ ID NO:5:
   Ser Xaa Xaa Xaa Xaa Arg Xaa Xaa Xaa Xaa Leu Xaa Xaa Xaa Xaa Xaa
   1               5                   10                  15
(2) INFORMATION FOR SEQ ID NO:6:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 2, 5, 7
      (D) OTHER INFORMATION: /note= Xaa is any amino acid
   (xi) SEQUENCE DESCRIPTION: SEQ ID NO:6:
   Pro Xaa Gly His Xaa Lys Xaa
   1               5
(2) INFORMATION FOR SEQ ID NO:7:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:7:
   Pro Pro Gly His Phe Lys Asp
   1               5
(2) INFORMATION FOR SEQ ID NO:8:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:8:
   Gly Ala Phe Pro Pro Gly His Phe Lys Asp Pro Asp Arg Leu
   1               5                   10
(2) INFORMATION FOR SEQ ID NO:9:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:9:
   Pro Arg Gly His Trp Lys Gln
   1               5
(2) INFORMATION FOR SEQ ID NO:10:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 5-7
      (D) OTHER INFORMATION: /note= Xaa is any amino acid
   (xi) SEQUENCE DESCRIPTION: SEQ ID NO:10:
   Pro Pro Gly His Xaa Xaa Xaa
   1               5
(2) INFORMATION FOR SEQ ID NO:11:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 3-7
      (D) OTHER INFORMATION: /note= Xaa is any amino acid
   (xi) SEQUENCE DESCRIPTION: SEQ ID NO:11:
   Pro Pro Xaa Xaa Xaa Xaa Xaa
   1               5
(2) INFORMATION FOR SEQ ID NO:12:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 5, 7
      (D) OTHER INFORMATION: /note= Xaa is Nal
   (xi) SEQUENCE DESCRIPTION: SEQ ID NO:12:
   Pro Pro Gly His Xaa Lys Xaa
   1               5
(2) INFORMATION FOR SEQ ID NO:13:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 5
      (D) OTHER INFORMATION: /note= Xaa is Nal
   (ix) FEATURE:
      (A) NAME/KEY: Modified-site
      (B) LOCATION: 7
      (D) OTHER INFORMATION: /note= Xaa is D-pF-Phe
   (xi) SEQUENCE DESCRIPTION: SEQ ID NO:13:
   Pro Pro Gly His Xaa Lys Xaa
   1               5
(2) INFORMATION FOR SEQ ID NO:14:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 7
      (D) OTHER INFORMATION: /note= Xaa is Nal
   (xi) SEQUENCE DESCRIPTION: SEQ ID NO:14:
   Pro Pro Gly His Phe Lys Xaa
   1               5
(2) INFORMATION FOR SEQ ID NO:15:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 5
      (D) OTHER INFORMATION: /note= Xaa is Nal
   (ix) FEATURE:
      (A) NAME/KEY: Modified-site
      (B) LOCATION: 7
      (D) OTHER INFORMATION: /note= Xaa is Abu
   (xi) SEQUENCE DESCRIPTION: SEQ ID NO:15:
   Pro Pro Gly His Xaa Lys Xaa
   1               5
(2) INFORMATION FOR SEQ ID NO:16:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 5
      (D) OTHER INFORMATION: /note= Xaa is Nal
   (ix) FEATURE:
      (A) NAME/KEY: Modified-site
      (B) LOCATION: 7
      (D) OTHER INFORMATION: /note= Xaa is D-Ala
   (xi) SEQUENCE DESCRIPTION: SEQ ID NO:16:
   Pro Pro Gly His Xaa Lys Xaa
   1               5
(2) INFORMATION FOR SEQ ID NO:17:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 3
      (D) OTHER INFORMATION: /note= Xaa is Pal
   (ix) FEATURE:
      (A) NAME/KEY: Modified-site
      (B) LOCATION: 4-7
      (D) OTHER INFORMATION: /note= Xaa is any amino acid
   (xi) SEQUENCE DESCRIPTION: SEQ ID NO:17: